(19) Europäisches Patentamt
European Patent Office
Office européen des brevets

(11) Numéro de publication: 0 239 461
A1

(12) # DEMANDE DE BREVET EUROPEEN

(21) Numéro de dépôt: **87400531.7**

(22) Date de dépôt: **11.03.87**

(51) Int. Cl.4: **C 07 D 211/46**, C 07 D 295/14, C 07 D 401/04, C 07 D 235/26, C 07 D 211/00, C 07 D 295/00, C 07 D 401/00

(30) Priorité: **12.03.86 FR 8603489**

(43) Date de publication de la demande: **30.09.87 Bulletin 87/40**

(84) Etats contractants désignés: **AT BE CH DE ES FR GB GR IT LI LU NL SE**

(71) Demandeur: **SYNTHELABO, 58 rue de la Glacière, F-75013 Paris (FR)**

(72) Inventeur: **Comarmond, Jacques, 10, Villa d'Este, F-75013 Paris (FR)**
Inventeur: **Purcell, Thomas, 47bis, rue de la Millière Les Mesnuls, F-78490 Montfort L'Amaury (FR)**
Inventeur: **Zard, Lydia, 6, Impasse des Quatre Vents La Croix Audierne, F-91190 Gif sur Yvette (FR)**

(74) Mandataire: **Ludwig, Jacques et al, SYNTHELABO Service Brevets 58, rue de la Glacière, F-75621 Paris Cedex 13 (FR)**

(54) **Dérivés de N-[[(hydroxy-2 phényl) (phényl) méthylène] amino-2]éthyl acétamide, leur préparation et leur application en thérapeutique.**

(57) Composé de formule

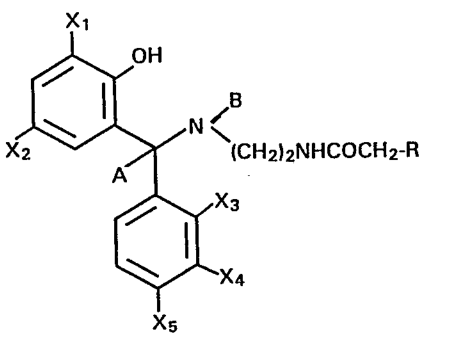

(I)

dans laquelle
A et B représentent chacun l'hydrogène ou forment ensemble une liaison, $X_1$ représente l'hydrogène ou un halogène ou un alkyle, $X_2$ et $X_5$ représentent chacun l'hydrogène ou un halogène ou un méthyle, $X_3$ représente l'hydrogène ou un halogène ou un hydroxyle, $X_4$ représente l'hydrogène ou un halogène, et R représente un morpholinyle-4, un pipéridinyle-1 ou un pipérazinyle-1 éventuellement substitués.

Application en thérapeutique.

EP 0 239 461 A1

La présente invention a pour objet des dérivés de N-[[(hydroxy-2 phényl)méthylène]amino-2 éthyl]acétamide, leur préparation et leur application en thérapeutique.

Les composés de l'invention répondent à la formule générale (I)

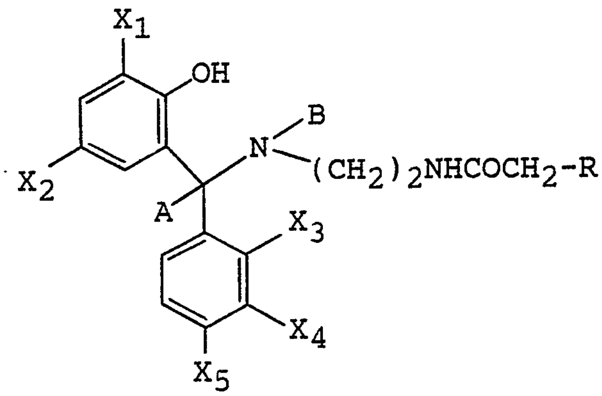

(I)

dans laquelle

A et B représentent chacun un atome d'hydrogène ou bien représentent ensemble une liaison carbone-azote,

$X_1$ représente un atome d'hydrogène ou d'halogène ou un groupe $(C_1-C_4)$alkyle,

$X_2$ représente un atome d'hydrogène ou d'halogène ou un groupe méthyle,

$X_3$ représente un atome d'hydrogène ou d'halogène ou un groupe hydroxyle,

$X_4$ représente un atome d'hydrogène ou d'halogène,

$X_5$ représente un atome d'hydrogène ou d'halogène ou un groupe méthyle, $(C_1-C_4)$alkylsulfonyle ou benzylsulfonyle éventuellement substitué en para par un atome d'halogène ou un groupe méthoxy, et

R représente un groupe morpholinyle-1, hydroxy-4 pipéridinyle-1, aminocarbonyl-4 pipéridinyle-1, hydroxy-4 phényl-4 pipéridinyle-1, (pipéridinyl-1)-4 pipéridinyle-1, (oxo-2 benzimidazolinyl-1)-4 pipéridinyle-1, pipérazinyle-1, méthyl-4 pipérazinyle-1, éthoxycarbonyl-4 pipérazinyle-1, phényl-4 pipérazinyle-1, chlorophényl-4 pipérazinyle-1, méthylphényl-4 pipérazinyle-1, ou (pyridinyl-2)-4 pipérazinyle-1.

Les sels d'addition que peuvent former les composés de l'invention avec des acides acceptables en pharmacologie, et les

divers isomères optiques, font partie de l'invention.
Les composés préférés de l'invention sont ceux de formule (I) dans laquelle R représente un groupe hydroxy-4 pipéridinyle-1, et A et B représentent ensemble une liaison carbone-azote.

Une autre catégorie de composés préférés est celle des composés de formule (I) dans laquelle $X_1$, $X_4$ et $X_5$ représentent chacun un atome d'hydrogène, $X_2$ représente un atome de chlore ou un groupe méthyle et $X_3$ représente un atome de chlore.

Les composés de l'invention peuvent être préparés selon le schéma ci-après.

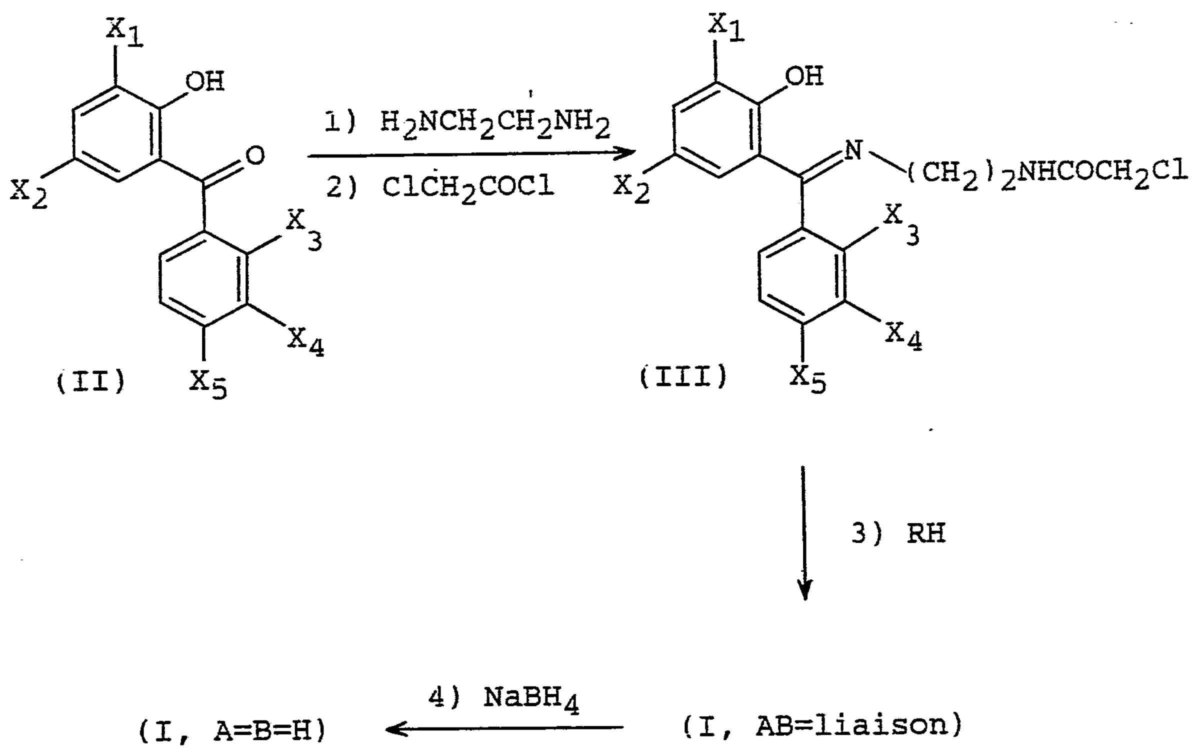

(II)
(III)
(I, A=B=H)
(I, AB=liaison)

On fait réagir d'abord une benzophénone de formule (II), dans laquelle $X_1$, $X_2$, $X_3$, $X_4$ et $X_5$ sont tels que définis ci-dessus, avec l'éthylènediamine dans une première étape, puis avec le chlorure de l'acide chloro-2 acétique dans une deuxième étape.
Les benzophénones (II) de départ sont connues ; elles sont décrites par exemple dans le brevet des Etats Unis d'Amérique n° 4 094 992 et dans le brevet britannique n° 2 111 051. Dans le cas où $X_5$ est un groupe alkylsulfonyle ou benzylsulfonyle

on peut, de manière connue, faire réagir le composé fluoré correspondant ($X_5$=F) avec un alkylmercaptan ou un benzylmercaptan, puis oxyder le produit obtenu, par exemple au moyen d'acide chloro-3 perbenzoïque.

La première étape du schéma ci-dessus a lieu de préférence avec un excès de diamine, dans un solvant tel que le chloroforme et à la température de reflux.

La deuxième étape a lieu dans le même récipient, à température ambiante, en présence d'une base destinée à fixer l'acide chlorhydrique libéré.

Dans une troisième étape on fait réagir le composé de formule (III) ainsi obtenu, avec une amine cyclique de formule RH, R étant tel que défini ci-dessus, à température ambiante ou avec un léger chauffage, dans un solvant tel qu'un alcool inférieur, et éventuellement en présence d'une base.

On obtient ainsi un composé de formule (I) dans laquelle A et B représentent ensemble une liaison carbone-azote.

Si l'on désire préparer un composé de formule (I) dans laquelle A et B représentent chacun un atome d'hydrogène, on fait subir au composé obtenu une réduction, par exemple au moyen d'un borohydrure de métal alcalin, à température ambiante et dans un solvant tel qu'un alcool inférieur.

Les exemples suivants illustrent en détail la préparation de quelques composés selon l'invention.

Les micro-analyses et les spectres IR et RMN ont confirmé la structure des produits obtenus.

Exemple 1 (Composé n° 1)

N-[[[(chloro-5 hydroxy-2 phényl)(chloro-2 phényl)méthylène] amino]-2 éthyl] (hydroxy-4 pipéridinyl-1)-2 acétamide.

a) Chloro-4 [(chloro-2 phényl)[(amino-2 éthyl)imino]]méthyl-2 phénol.

On introduit 53,4 g (0,2 mole) de (chloro-5 hydroxy-2 phényl) (chloro-2 phényl)méthanone dans 500 ml de chloroforme, on ajoute 140 ml (2,1 mole) de diamino-1,2 éthane et on chauffe au reflux pendant 2 h. On lave ensuite la phase organique à l'eau jusqu'à obtenir un pH de 7 à 8.

b) Chloro-2 N-[[[(chloro-5 hydroxy-2 phényl)(chloro-2 phényl) méthylène]amino]-2 éthyl]-acétamide.

A la solution précédemment obtenue on ajoute 18 g (0,2 mole) de bicarbonate de sodium, 100 ml d'eau et 16 ml (0,2 mole) de chlorure de chloroacétyle. On agite le mélange pendant 30 mn à température ambiante, on sépare et sèche la phase organique, et on évapore le solvant.

On reprend le résidu à chaud avec du méthanol, on décolore la solution avec du noir animal. On la laisse refroidir, et on filtre les cristaux formés. On obtient 43 g d'un produit jaune. Point de fusion : 122-124°C.

c) N-[[[(chloro-5 hydroxy-2 phényl)(chloro-2 phényl) méthylène]amino]-2 éthyl] (hydroxy-4 pipéridinyl-1)-2 acétamide.

On introduit 9 g (0,023 mole) du dérivé précédemment obtenu dans 100 ml d'éthanol contenant 5 g (0,05 mole) de hydroxy-4 pipéridine. On agite 1 h à température ambiante, on évapore l'alcool et on reprend le résidu avec un mélange eau/acétate d'éthyle. On sépare la phase organique, on la sèche sur sulfate de magnésium, on chasse le solvant et on recristallise le résidu dans l'alcool isopropylique. On obtient 10,8 g de produit. Point de fusion : 154-155°C.

Exemple 2 (Composé n° 7)

N-[[[(méthyl-5 hydroxy-2 phényl)(chloro-2 phényl)méthylène] amino]-2 éthyl] (hydroxy-4 pipéridinyl-1)-2 acétamide.

a) Méthyl-4 [(chloro-2 phényl)[amino-2 éthyl)imino]]méthyl-2 phénol.

On introduit 12,3 g (0,05 mole) de (méthyl-5 hydroxy-2 phényl)(chloro-2 phényl)méthanone dans 150 ml de chloroforme, on ajoute 35 ml (0,5 mole) de diamino-1,2 éthane, et on chauffe au reflux pendant 3 h. Puis on lave la phase organique trois fois à l'eau.

b) Chloro-2 N-[[[(méthyl-5 hydroxy-2 phényl)(chloro-2 phényl) méthylène]amino]-2 éthyl]-acétamide.

A la solution précédemment obtenue on ajoute 30 ml d'eau contenant 4,65 g (0,055 mole) de bicarbonate de sodium, puis 4,11 ml (0,051 mole) de chlorure de chloroacétyle. On agite le mélange à température ambiante, on sépare la phase organique, on la sèche sur sulfate de magnésium et on évapore le solvant. On fait cristalliser le résidu dans un mélange d'acétate d'éthyle et d'éther isopropylique. On recueille 13 g de produit. Point de fusion : 94-95°C.

c) N-[[[(méthyl-5 hydroxy-2 phényl)(chloro-2 phényl) méthylène] amino]-2 éthyl] (hydroxy-4 pipéridinyl-1)-2 acétamide.

On introduit 7,25 g (0,02 mole) du dérivé précédemment obtenu dans 150 ml d'éthanol contenant 4 g (0,04 mole) de hydroxy-4 pipéridine. On agite le mélange pendant une journée à température ambiante, on évapore l'alcool et on purifie le résidu par chromatographie sur silice en éluant avec un mélange 90/10 de dichlorométhane/méthanol ; on fait recristalliser le produit obtenu dans l'éther éthylique. On en recueille 6 g. Point de fusion : 139-140°C.

Exemple 3 (Composé n° 18)

N-[[[(chloro-5 hydroxy-2 phényl)(chloro-2 phényl)méthylène] amino]-2 éthyl][(chloro-4 phényl)-4 pipérazinyl-1]-2 acétamide.

On dissout 10 g (0,026 mole) de chloro-2 N-[[[(chloro-5 hydroxy-2 phényl)(chloro-2 phényl) méthylène]amino]-2 éthyl]-acétamide obtenu selon l'exemple 1, a) et b), dans 200 ml d'éthanol et on ajoute 15,5 g (0,057 mole) de dichlorhydrate de (chloro-4 phényl)-1 pipérazine, tout en agitant le mélange à température ambiante. Ensuite on ajoute 9 g de carbonate de sodium et on chauffe le mélange au bain-marie à 50°C pendant 3 h. On isole la phase organique, on évapore l'alcool et on purifie le résidu par chromatographie sur silice en éluant avec un mélange 97,5/2,5 de dichlorométhane/méthanol. On recueille finalement 11,4 g de produit pur, sous forme de mousse. Point de fusion : 78-80°C.

Exemple 4 (Composé n° 20)

N-[[[(chloro-5 hydroxy-2 phényl)(chloro-2 phényl)méthylène] amino]-2 éthyl][(pyridinyl-2)-4 pipérazinyl-1]-2 acétamide.

On dissout 10 g (0,026 mole) de chloro-2 N-[[[(chloro-5 hydroxy-2 phényl)(chloro-2 phényl) méthylène]amino]-2 éthyl]-acétamide obtenu selon l'exemple 1, a) et b), dans 200 ml d'éthanol et, sous agitation à température ambiante, on ajoute 9,4 g (0,057 mole) de (pyridinyl-2)-1 pipérazine. On chauffe le mélange en bain-marie à 50°C pendant 3 h. On isole la phase organique, on évapore l'alcool et on purifie le résidu par chromatographie sur silice en éluant avec un mélange 97,5/2,5 de dichlorométhane/méthanol.
On recueille finalement 13 g de produit pur, sous forme de mousse. Point de fusion : 68-71°C.

Exemple 5 (Composé n° 28)
N-[[[(chloro-5 hydroxy-2 phényl)(chloro-2 phényl)méthyl] amino]-2 éthyl][(pyridinyl-2)-4 pipérazinyl-1]-2 acétamide.

On dissout 4,6 g (0,0089 mole) de N-[[[(chloro-5 hydroxy-2 phényl)(chloro-2 phényl)méthylène]amino]-2 éthyl][(pyridinyl-2)-4 pipérazinyl-1]-2 acétamide, obtenu selon l'exemple 4, dans 100 ml d'éthanol, on ajoute 1 g (3 équivalents) de borohydrure de sodium, on agite le mélange pendant 1 h à température ambiante, et on le laisse reposer une nuit. On évapore l'alcool sous vide, on reprend le résidu avec du dichlorométhane, on lave le mélange deux fois à l'eau, on le filtre, on le sèche sur sulfate de magnésium et on évapore le solvant. Il reste une huile qu'on purifie par chromatographie sur silice en éluant avec un mélange 95/5 de dichlorométhane/méthanol. On recueille finalement 4 g de produit pur sous forme de mousse. Point de fusion : 72-75°C.

Le tableau suivant illustre les stuctures et propriétés physiques de quelques composés selon l'invention.

Tableau

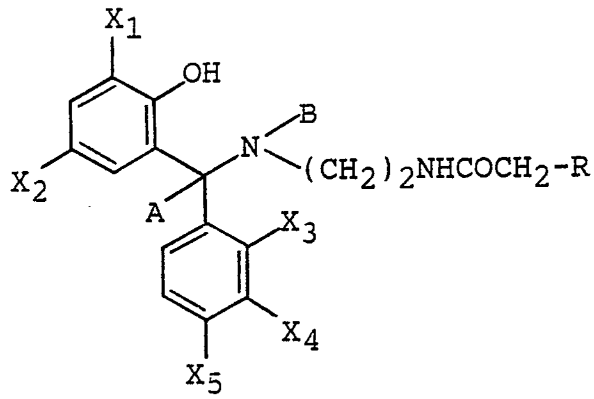

(I)

| N° | AB | $X_1$ | $X_2$ | $X_3$ | $X_4$ | $X_5$ | R | F(°C) |
|---|---|---|---|---|---|---|---|---|
| 1 | — | H | Cl | Cl | H | H | 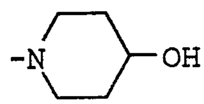 | 154-155 |
| 2 | — | H | Cl | H | H | H | 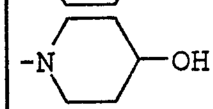 | 134-135 |
| 3 | — | $CH_3$ | Cl | H | H | Cl | 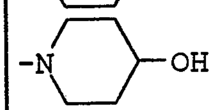 | 114-115 |
| 4 | — | H | Cl | H | H | $CH_3$ | 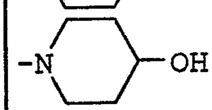 | 158-159 |
| 5 | — | H | Cl | H | Cl | Cl | 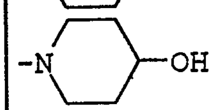 | 130-131 |
| 6 | — | Cl | Cl | Cl | H | H | 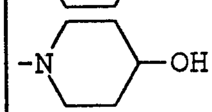 | 125-126 |
| 7 | — | H | $CH_3$ | Cl | H | H | 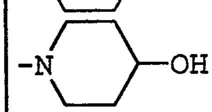 | 139-140 |
| 8 | — | H | Cl | Cl | H | Cl | 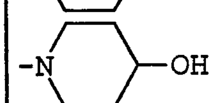 | 153-154 |
| 9 | — | H | Cl | H | H | Cl | 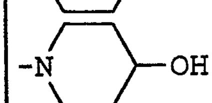 | 150-152 |
| 10 | — | $nC_3H_7$ | Cl | H | H | Cl | 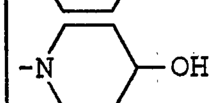 | (2) |
| 11 | — | H | H | OH | H | H | 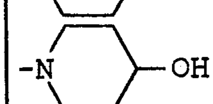 | 82-86(2) |
| 12 | — | H | Cl | F | H | H | 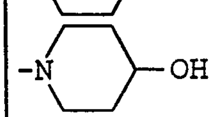 | 153-155 |

| N° | AB | $X_1$ | $X_2$ | $X_3$ | $X_4$ | $X_5$ | R | F(°C) |
|---|---|---|---|---|---|---|---|---|
| 13 | — | H | Cl | Cl | H | H | 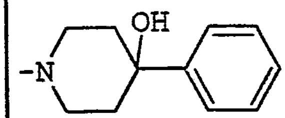 | ≈88(1) |
| 14 | — | H | Cl | Cl | H | H | 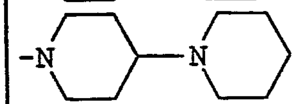 | (1) |
| 15 | — | H | Cl | Cl | H | H | 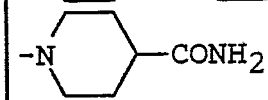 | (1) |
| 16 | — | H | Cl | Cl | H | H | 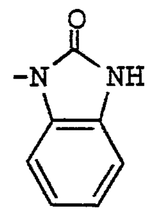 | ≈170(1) |
| 17 | — | H | Cl | Cl | H | H | 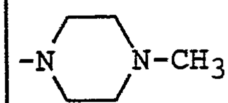 | (1) |
| 18 | — | H | Cl | Cl | H | H | 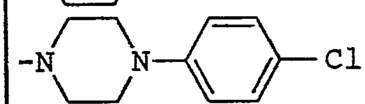 | 78-80(2) |
| 19 | — | H | Cl | Cl | H | H | 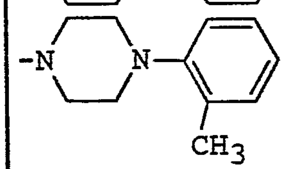 | 68-71(2) |
| 20 | — | H | Cl | Cl | H | H | 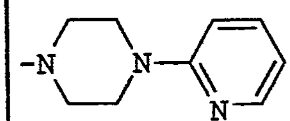 | 68-71(2) |
| 21 | — | H | Cl | Cl | H | H | 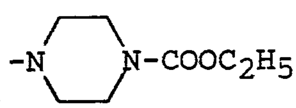 | 60-64(2) |
| 22 | — | H | Cl | Cl | H | H | 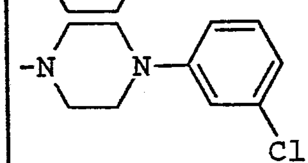 | 68-69(2) |
| 23 | — | H | Cl | Cl | H | H | 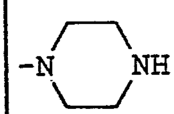 | 84-88(2) |
| 24 | — | H | Cl | Cl | H | H | 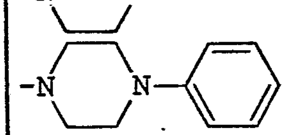 | 113-117 |
| 25 | — | H | F | Cl | H | H | 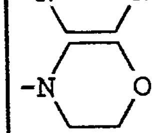 | 103-105 |

| N° | AB | $X_1$ | $X_2$ | $X_3$ | $X_4$ | $X_5$ | R | F(°C) |
|---|---|---|---|---|---|---|---|---|
| 26 | HH | H | Cl | Cl | H | H | 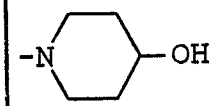 | 152-154 |
| 27 | HH | H | Cl | Cl | H | H | 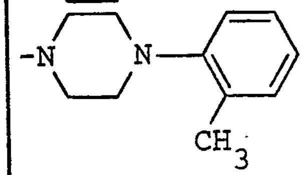 | 71-73(2) |
| 28 | HH | H | Cl | Cl | H | H | 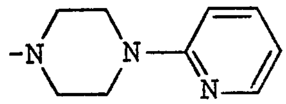 | 72-75(2) |
| 29 | HH | H | Cl | Cl | H | H | 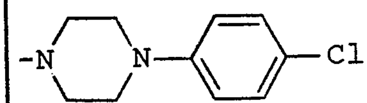 | 76-79(2) |
| 30 | HH | H | Cl | Cl | H | H | 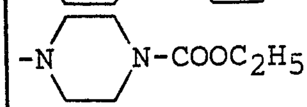 | 70-71(2) |
| 31 | HH | H | Cl | Cl | H | H | 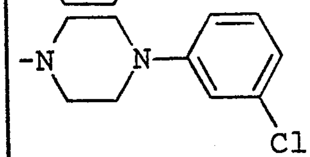 | 78-80(2) |
| 32 | — | Cl | Cl | H | H | $SO_2CH_2C_6H_5$ | 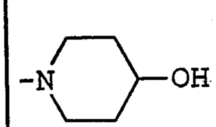 | 173-175 |
| 33 | — | Cl | Cl | H | H | $SO_2CH_2C_6H_4Cl$ | 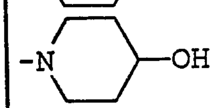 | 162-165 |
| 34 | — | Cl | Cl | H | H | $SO_2CH_2C_6H_4OCH_3$ | 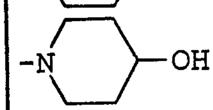 | 213-215 |
| 35 | — | $CH_3$ | Cl | H | H | $SO_2CH_2C_6H_5$ | 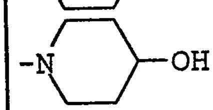 | 202-203 |
| 36 | — | Cl | Cl | H | H | $SO_2iC_3H_7$ | 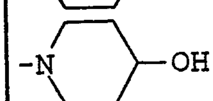 | 105-106 |
| 37 | — | $CH_3$ | Cl | H | H | $SO_2CH_2C_6H_5$ | 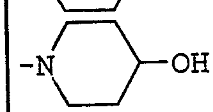 | 164-166 |
| 38 | HH | Cl | Cl | H | H | $SO_2CH_2C_6H_5$ | 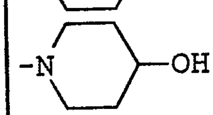 | 139-141 |

Notes : les composés sont sous forme de bases libres

(1) huile vitrifiée

(2) formation d'une mousse.

Les composés de l'invention ont fait l'objet d'essais toxicologiques et pharmacologiques qui ont mis en évidence leur intérêt comme substances actives de médicaments.

Leur toxicité aigüe est faible : la dose léthale à 50 % ($LD_{50}$) déterminée chez la souris est supérieure à 1000 mg/kg par la voie orale pour la plupart d'entre eux.

Les composés de l'invention ont été testés quant à leur effet sur l'ulcère d'estomac induit par la phénylbutazone chez le rat.

Le test est effectué sur des rats Wistar femelles pesant 180-210 g à jeun depuis 20 heures, répartis en blocs randomisés.
Les ulcères sont provoqués par l'administration orale de phénylbutazone en solution mole à mole avec de la soude, à la dose de 200 mg/kg.
Les composés à étudier sont administrés par voie orale à raison de 10, 30 ou 100 mg/kg, 30 minutes avant l'ingestion de phénylbutazone, les animaux témoins ne recevant que le placébo.
Deux heures après l'administration de l'agent ulcérigène, les animaux sont sacrifiés par inhalation de chloroforme. Les estomacs sont prélevés et le degré d'ulcération est noté en aveugle de 0 à 3.

Le tableau suivant donne les toxicités et les diminutions maximales d'ulcères obtenues aux doses indiquées, pour quelques composés de l'invention.
Il montre que ces derniers ont une activité antiulcéreuse significative.

Tableau

| Composé n° | $DL_{50}$ (mg/kg) p.o. | Diminution ulcération (%) | Dose (mg/kg) p.o. |
|---|---|---|---|
| 1 | >1000 | 100 | 30 |
| 2 | >1000 | 100 | 100 |
| 3 | >1000 | 94 | 30 |
| 4 | >1000 | 96 | 100 |
| 5 | >1000 | 92 | 30 |
| 6 | >1000 | 100 | 30 |
| 7 | >1000 | 95 | 30 |
| 8 | >1000 | 98 | 100 |
| 16 | >1000 | 64 | 30 |
| 19 | >1000 | 71 | 100 |
| 23 | 100>>300 | 87 | 10 |
| 26 | >1000 | 97 | 30 |
| 27 | >1000 | 98 | 30 |
| 29 | >1000 | 98 | 30 |

D'autres essais ont montré que certains composés de l'invention possèdent également des propriétés antidépressives et analgésiques. Ils peuvent donc être utilisés pour le traitement de la dépression, de douleurs d'origines diverses (par exemple post-chirurgicale, dentaire, migraines...), et des ulcères gastriques, duodénaux ou gastroduodénaux.

Les composés de l'invention peuvent être administrés par voie orale ou parentérale, sous la forme de compositions pharmaceutiques contenant la substance active en association avec tout excipient approprié, par exemple sous la forme de comprimés, dragées, gélules, capsules ou de solutions ou de suspensions buvables ou injectables.

La posologie quotidienne peut aller de 10 à 2000 mg.

Revendications pour les états contractants : BE, CH, DE, FR, GB, IT, LI, LU, NL, SE.

1. Composé, sous forme d'isomère optique pur ou de mélange d'isomères optiques, caractérisé en ce qu'il répond à la formule générale (I)

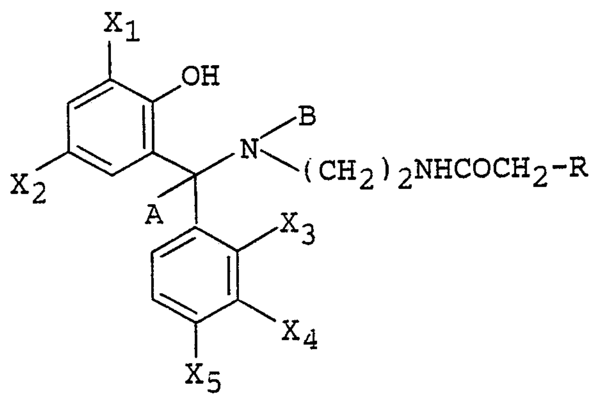

(I)

dans laquelle

A et B représentent chacun un atome d'hydrogène ou bien représentent ensemble une liaison carbone-azote,

$X_1$ représente un atome d'hydrogène ou d'halogène ou un groupe $(C_1-C_4)$alkyle

$X_2$ représente un atome d'hydrogène ou d'halogène ou un groupe méthyle,

$X_3$ représente un atome d'hydrogène ou d'halogène ou un groupe hydroxyle,

$X_4$ représente un atome d'hydrogène ou d'halogène,

$X_5$ représente un atome d'hydrogène ou d'halogène ou un groupe méthyle, $(C_1-C_4)$alkylsulfonyle ou benzylsulfonyle éventuellement substitué en para par un atome d'halogène ou un groupe méthoxy, et

R représente un groupe morpholinyle-1, hydroxy-4 pipéridinyle-1, aminocarbonyl-4 pipéridinyle-1, hydroxy-4 phényl-4 pipéridinyle-1, (pipéridinyl-1)-4 pipéridinyle-1, (oxo-2 benzimidazolinyl-1)-4 pipéridinyle-1, pipérazinyle-1, méthyl-4 pipérazinyle-1, éthoxycarbonyl-4 pipérazinyle-1, phényl-4 pipérazinyle-1, chlorophényl-4 pipérazinyle-1, méthylphényl-4 pipérazinyle-1, ou (pyridinyl-2)-4 pipérazinyle-1, ainsi que ses sels d'addition à des acides acceptables en pharmacologie.

2. Composé selon la revendication 1, caractérisé en ce que A et B représentent ensemble une liaison carbone-azote, et R représente un groupe hydroxy-4 pipéridinyle.

3. Composé selon l'une des revendications 1 et 2, caractérisé en ce que $X_1$, $X_4$ et $X_5$ représentent chacun un atome d'hydrogène, $X_2$ représente un atome de chlore ou un groupe méthyle et $X_3$ représente un atome de chlore.

4. Procédé de préparation d'un composé tel que défini dans la revendication 1, procédé caractérisé en ce qu'on fait réagir une benzophénone de formule (II)

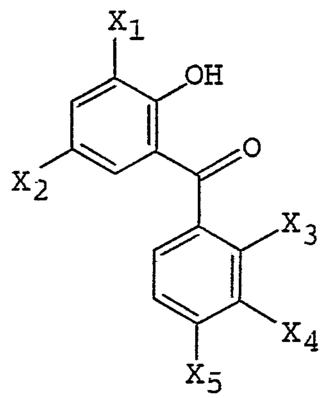

(II)

dans laquelle $X_1$, $X_2$, $X_3$, $X_4$ et $X_5$ sont tels que définis dans la revendication 1, avec un excès d'éthylènediamine, dans un solvant tel que le chloroforme et à température de reflux, puis on fait réagir le composé obtenu avec le chlorure d'acide chloro-2 acétique à température ambiante et en présence d'une base, puis on fait réagir le composé obtenu, de formule (III)

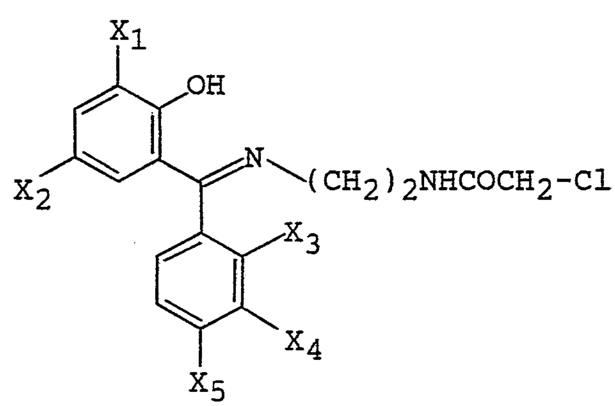

(III)

avec une amine de formule RH, dans laquelle R est tel que

défini dans la revendication 1, à température ambiante ou avec un léger chauffage, dans un solvant tel qu'un alcool inférieur et éventuellement en présence d'une base, et, si dans la formule (I) A et B représentent chacun un atome d'hydrogène, on soumet le composé obtenu à une réduction au moyen d'un borohydrure de métal alcalin, à température ambiante et dans un solvant tel qu'un alcool inférieur.

5. Médicament, caractérisé en ce qu'il consiste en un composé selon l'une quelconque des revendications 1 à 3.

6. Composition pharmaceutique, caractérisée en ce qu'elle contient un composé selon l'une quelconque des revendications 1 à 3, associé à un excipient pharmaceutique.

Revendication pour les états contractants : AT, ES, GR.

Procédé de préparation d'un composé répondant à la formule générale (I)

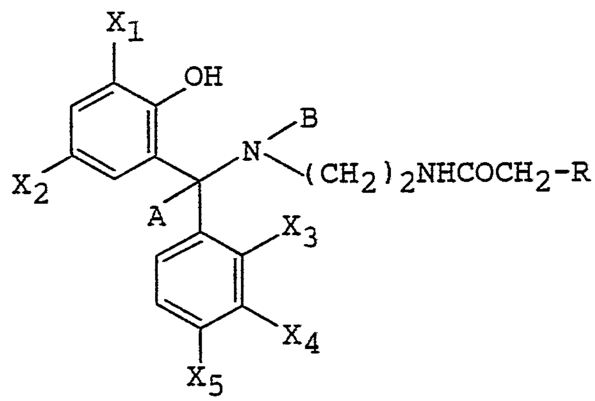

(I)

dans laquelle

A et B représentent chacun un atome d'hydrogène ou bien représentent ensemble une liaison carbone-azote,

$X_1$ représente un atome d'hydrogène ou d'halogène ou un groupe $(C_1-C_4)$alkyle

$X_2$ représente un atome d'hydrogène ou d'halogène ou un groupe méthyle,

$X_3$ représente un atome d'hydrogène ou d'halogène ou un groupe hydroxyle,

$X_4$ représente un atome d'hydrogène ou d'halogène,

$X_5$ représente un atome d'hydrogène ou d'halogène ou un groupe méthyle, $(C_1-C_4)$alkylsulfonyle ou benzylsulfonyle éventuellement substitué en para par un atome d'halogène ou un groupe méthoxy, et

R représente un groupe morpholinyle-1, hydroxy-4 pipéridinyle-1, aminocarbonyl-4 pipéridinyle-1, hydroxy-4 phényl-4 pipéridinyle-1, (pipéridinyl-1)-4 pipéridinyle-1, (oxo-2 benzimidazolinyl-1)-4 pipéridinyle-1, pipérazinyle-1, méthyl-4 pipérazinyle-1, éthoxycarbonyl-4 pipérazinyle-1, phényl-4 pipérazinyle-1, chlorophényl-4 pipérazinyle-1, méthylphényl-4 pipérazinyle-1, ou (pyridinyl-2)-4 pipérazinyle-1,

procédé caractérisé en ce qu'on fait réagir une benzophénone de formule (II)

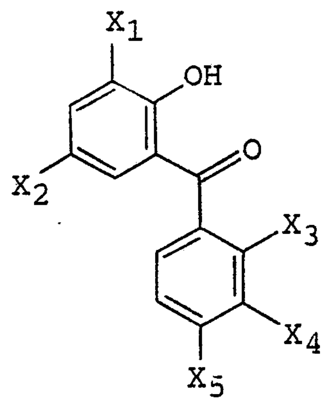

(II)

dans laquelle $X_1$, $X_2$, $X_3$, $X_4$ et $X_5$ sont tels que définis ci-dessus, avec un excès d'éthylènediamine, dans un solvant tel que le chloroforme et à température de reflux, puis on fait réagir le composé obtenu avec le chlorure d'acide chloro-2 acétique à température ambiante et en présence d'une base, puis on fait réagir le composé obtenu, de formule (III)

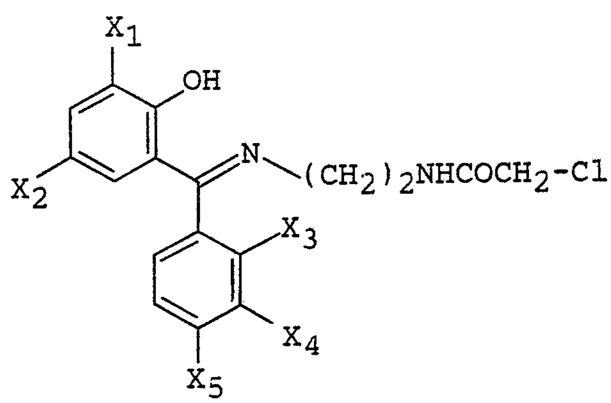

(III)

avec une amine de formule RH, dans laquelle R est tel que défini ci-dessus, à température ambiante ou avec un léger chauffage, dans un solvant tel qu'un alcool inférieur et éventuellement en présence d'une base, et, si dans la formule (I) A et B représentent chacun un atome d'hydrogène, on soumet le composé obtenu à une réduction au moyen d'un borohydrure de métal alcalin, à température ambiante et dans un solvant tel qu'un alcool inférieur.

0239461

Office européen des brevets

# RAPPORT DE RECHERCHE EUROPEENNE

Numero de la demande

EP 87 40 0531

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | DIE PHARMAZIE, vol. 35, no. 4, avril 1980, pages 256-263; F. BAHR et al.: "Zur Herstellung und Umwandlung von 3,4-Dihydrochinazolin-4-olen 1,2" * Page 259, schéma 5, composé 1-G * --- | 1-6 | C 07 D 211/46<br>C 07 D 295/14<br>C 07 D 401/04<br>C 07 D 235/26 |
| D,Y | GB-A-2 111 051 (SYNTHELABO) * En entier * --- | 1-6 | |
| D,Y | US-A-4 094 992 (J.P. KAPLAN) * En entier * ----- | 1-6 | |
| | | | DOMAINES TECHNIQUES RECHERCHES (Int. Cl.4) |
| | | | C 07 D 211/00<br>C 07 D 295/00<br>C 07 D 401/00 |

Le présent rapport de recherche a été établi pour toutes les revendications

| Lieu de la recherche | Date d'achèvement de la recherche | Examinateur |
|---|---|---|
| LA HAYE | 17-06-1987 | MAISONNEUVE J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03 82